(19) Europäisches Patentamt
European Patent Office
Office européen des brevets

(11) Veröffentlichungsnummer : **0 151 368 B2**

# (12) NEUE EUROPÄISCHE PATENTSCHRIFT

(45) Veröffentlichungstag der neuen Patentschrift :
**15.09.93 Patentblatt 93/37**

(51) Int. Cl.$^5$ : **C07C 67/03,** C07C 69/732

(21) Anmeldenummer : **84810631.6**

(22) Anmeldetag : **17.12.84**

## (54) Verfahren zur Herstellung eines Hydroxyphenylcarbonsäureesters.

Die Akte enthält technische Angaben, die nach dem Eingang der Anmeldung eingereicht wurden und die nicht in dieser Patentschrift enthalten sind.

(30) Priorität : **22.12.83 CH 6858/83**

(43) Veröffentlichungstag der Anmeldung :
**14.08.85 Patentblatt 85/33**

(45) Bekanntmachung des Hinweises auf die Patenterteilung :
**30.08.89 Patentblatt 89/35**

(45) Bekanntmachung des Hinweises auf die Entscheidung über den Einspruch :
**15.09.93 Patentblatt 93/37**

(84) Benannte Vertragsstaaten :
**DE FR GB IT NL**

(56) Entgegenhaltungen :
**EP-A- 0 032 459**
**EP-A- 0 066 189**
**EP-A- 0 102 920**
**DE-A- 1 543 644**
**DE-A- 2 240 609**
**US-A- 3 644 482**
**US-A- 4 228 297**

(73) Patentinhaber : **CIBA-GEIGY AG**
**Klybeckstrasse 141**
**CH-4002 Basel (CH)**

(72) Erfinder : **Gubler, Erich**
**Salmenstrasse 4**
**CH-4127 Birsfelden (CH)**
Erfinder : **Siegrist, Max, Dr.**
**Römerstrasse 45**
**CH-4415 Lausen (CH)**

**EP 0 151 368 B2**

## Beschreibung

Die vorliegende Erfindung betrifft ein Verfahren zur Herstellung von kristallinem Pentaerythrityl-tetrakis-[3-(3,5-di-t-butyl-4-hydroxyphenyl)-propionat] durch Umesterung von überschüssigem Niederalkyl-3-(3,5-di-t-butyl-4-hydroxyphenyl)-propionat mit Pentaerythrit in Abwesenheit eines inerten Lösungsmittels.

Ein Verfahren zur Herstellung von Pentaerythrityl-tetrakis-[3-(3,5-di-t-butyl-4-hydroxyphenyl)-propionat] ausgehend von 2,6-Di-t-butylphenol und Methacrylat über Methyl-3-(3,5-di-t-butyl-4-hydroxyphenyl)-propionat und dessen Umesterung mit Pentaerythrit in Tetralin ist im US-Patent 4 228 297 beschrieben. Die Ausbeute ist jedoch bei diesem Verfahren unbefriedigend. Ein weiteres Verfahren zur Herstellung von Pentaerythrityl-tetrakis-[3-(3,5-di-t-butyl-4-hydroxyphenyl)-propionat] ist im US-Patent 3 644 482 beschrieben. Dabei wird bei der Umesterung von Methyl-3-(3,5-di-t-butyl-4-hydroxyphenyl)-propionat mit Pentaerythrit kein inertes Lösungsmittel jedoch ein Ueberschuss an Ester von 9,1 % eingesetzt. In der allgemeinen Beschreibung wird die Zweckmässigkeit eines 5 bis 10 %igen Ueberschusses an Ester dargelegt. Das Verfahren liefert eine glasige Substanz, ebenfalls mit unbefriedigender Ausbeute.

Es wurde nun gefunden, dass beim Durchführen der Umesterung mit einem Ueberschuss an Niederalkyl-3-(3,5-di-t-butyl-4-hydroxyphenyl)-propionat von 25 bis 35 % der stöchiometrischen Menge von 4 Mol auf 1 Mol Pentaerythrit ohne Zusatz eines inerten Lösungsmittels, überraschenderweise kristallines Pentaerythrityl-tetrakis-[3-(3,5-di-t-butyl-4-hydroxyphenyl)-propionat] mit ausgezeichneten Ausbeuten erhalten wird.

Die vorliegende Erfindung betrifft demnach ein Verfahren zur Herstellung von Pentaerythrit-tetrakis-[3-(3,5-di-t-butyl-4-hydroxyphenyl)-propionat] durch Umesterung eines $C_1$-$C_4$ Alkyl-3-(3,5-di-t-butyl-4-hydroxyphenyl)-propionats mit Pentaerythrit in Gegenwart katalytischer Mengen eines basischen Katalysators, dadurch gekennzeichnet, dass die Umesterung bei einem Ueberschuss an $C_1$-$C_4$ Alkyl-3-(3,5-di-t-butyl-4-hydroxyphenyl)-propionat von 25 bis 35 %, der stöchiometrischen Menge von 4 Mol auf 1 Mol Pentaerythrit durchgeführt wird.

$C_1$-$C_4$ Alkyl bedeutet z. B. Methyl, Ethyl, Propyl, Isopropyl, n-Butyl. Bevorzugt ist Methyl.

Der Katalysator wird z. B. in Mengen zwischen 0,5 und 10 Mol %, bevorzugt zwischen 1,0 und 6 Mol %, bezogen auf das Pentaerythrit eingesetzt.

Geeignete Katalysatoren sind die für Umesterungen üblichen basischen Katalysatoren, z. B. Alkaliamide, wie Lithiumamid, Alkalihydride, wie Lithiumhydrid, Alkalihydroxide, wie Kalium- oder Natriumhydroxid, Alkalialkoholate, wie Natrium-methylat oder -ethylat, sowohl als auch die Oxide von Metallen der vierten Haupt- oder Nebengruppe des Periodensystems, wie Germanium- und Zirkondioxid, metallorganische Verbindungen eines Metalls der vierten Haupt- oder Nebengruppe des Periodensystems wie solche der Formel I

$$(R_1O)_{\overline{4}}M \quad (I)$$

worin $R_1$ $C_1$-$C_{18}$ Alkyl, Phenyl oder Benzyl bedeutet und M das Element Ge, Zr oder Sn ist, oder solche der Formel II

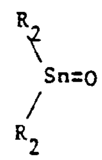

worin $R_2$ $C_4$-$C_{12}$ Alkyl bedeutet.

$R_2$ bedeutet als $C_4$-$C_{12}$ Alkyl z. B. n-Butyl, n-Pentyl, n-Hexyl, n-Octyl, n-Decyl, n-Dodecyl.

$R_1$ hat als $C_1$-$C_{18}$ Alkyl die gleiche Bedeutung wie $R_2$ und ist zusätzlich z. B. auch Methyl, Ethyl, Propyl, Isopropyl, n-Hexadecyl und n-Octadecyl.

$R_1$ und $R_2$ sind bevorzugt n-Butyl.

Bevorzugte Katalysatoren sind Dibutylzinnoxid und insbesondere Lithiumamid.

Die Umesterung wird vorteilhaft bei einer Temperatur zwischen 120 und 220 °C, bevorzugt zwischen 140 und 200 °C, und bei einem Druck zwischen 10 und 2 mbar, durchgeführt.

Das Verfahren kann beispielsweise wie folgt ausgeführt werden: Der bei der Umesterung entstehende $C_1$-$C_4$ Alkyl-alkohol wird durch Destillation unter Vakuum entfernt. Das überschüssige $C_1$-$C_4$ Alkyl-3-(3,5-di-t-butyl-4-hydroxyphenyl)-propionat wird am Ende der Reaktion unter Vakuum abdestilliert (es kann beim nächsten Ansatz wieder eingesetzt werden) und das anfallende Pentaerthrityl-tetrakis-[3-(3,5-di-t-butyl-4-hydroxyphenyl)-propionat] als heisse Schmelze klärfiltriert und anschliessend nach üblichen Methoden, bevorzugt aus einem mit Essigsäure leicht angesäuertem $C_1$-$C_4$ Alkyl-alkohol, insbesondere Methanol, kristallisiert.

Bei den Ausgangsprodukten und Katalysatoren handelt es sich um bekannte, im Handel erhältliche Substanzen.

Das erfindungsgemässe Verfahren zeichnet sich durch die hervorragend gute Ausbeute an einem reinen kristallinen Produkt von ausserordentlicher Rieselfähigkeit aus. Ein weiterer Vorteil dieses Verfahrens ist die Lösungsmittel-freie Arbeitsweise.

Pentaerythrityl-tetrakis-[3-(3,5-di-t-butyl-4-hydroxyphenyl)-propionat] ist ein bekannter Stabilisator für organische Materialien, die der Zersetzung unterworfen sind, zum Beispiel für synthetische organische Polymere, tierische und pflanzliche Oele, Kohlenwasserstoffe, Schmiermittel und dergleichen.

Die folgenden Beispiele erläutern die Erfindung, ohne sie einzuschränken.

Beispiel 1

In einem 6.300 Liter Kessel werden 5 010 kg (17,15 kMol = 30 Mol % Überschuss bezogen auf Pentaerythrit) Methyl-3-(3,5-di-t-butyl-4-hydroxyphenyl)-propionat (im folgenden kurz Methylester genannt) als Schmelze von ca. 80 °C, 450 kg (3.3 kMol) Pentaerythrit und 3 kg (0,13 kMol = 4 Mol % bezogen auf Pentaerythrit) Lithiumamid vorgelegt und dann unter Rühren und bei einem Druck von ca. 200 mbar innert 100 Minuten auf 158 °C erhitzt. Bei 120-125 °C beginnt Methanol in eine Vorlage abzudestillieren. Sobald ca. 380 Liter Methanol abdestilliert sind, wird das Destillationssystem auf Rückfluss gestellt, das Vakuum voll geöffnet und auf ca. 200 °C weitergeheizt. Bei ca. 175 °C und 80 mbar beginnt Methylester unter Rückfluss zu sieden. Es wird dann allmählich ein Vakuum von 7 bis 10 mbar und eine Temperatur von 198 °C erreicht, wobei freiwerdendes Methanol via Vakuumpumpe entfernt wird. Der Methylesterrückfluss wird eine halbe Stunde gehalten und anschliessend der Methylesterüberschuss in eine Vorlage abdestilliert. Am Schluss der Destillation, d.h. nach etwa 8 Stunden, wird ein Vakuum von 3 bis 5 mbar, eine Temperatur von 200 °C und eine Methylestermenge von 1'080 kg erreicht.

Der abdestillierte Methylester wird in einem weiteren Ansatz wieder eingesetzt.

Die heisse Reaktionsschmelze wird bei 180 bis 200 °C klärfiltriert und anschliessend in mit Essigsäure leicht angesäuertem Methanol eingetragen und auskristallisiert.

Man erhält 3 700 kg (95,0 % d. Th.) kristallines Pentaerythrityl-tetrakis-[3-(3,5-di-butyl-4-hydroxyphenyl)-propionat] mit Smp. 118-119 °C.

Vergleichsbeispiele 2 und 3

Das Verfahren von Beispiel 1 wird wiederholt mit der einzigen Ausnahme, dass der Ueberschuss an Methylester wie in nachstehender Tabelle 1 angegeben variiert wird.

**Tabelle 1**

| Vergleichs-Beispiel | Mol % Methylester-Ueberschuss bezogen auf Pentaerythrit | Ausbeute | Smp. |
|---|---|---|---|
| 2 | 20 | 88,3 | 114 |
| 3 | 50 | 91,9 | 114 |

Vergleichsbeispiele 4-10

Das Verfahren von Beispiel 1 wird wiederholt mit anderen Katalysatoren in unterschiedlichen Mengenverhältnissen bei verschiedenen Temperaturen, Drücken und Methylester-Ueberschüssen, wie in nachstehender Tabelle 2 aufgezeichnet.

Tabelle 2

| Beispiel | Katalysator | Mol % Katalysator bezogen auf Pentaerythrit | Temperatur (°C) | Druck (mbar) | Mol % Methylester bezogen auf Pentaerythrit | Ausbeute | Smp. (°C) |
|---|---|---|---|---|---|---|---|
| 4 | Lithiumhydrid | 2,5 | 150 | 3 | 45 | 90,9 | 114 |
| 5 | Lithiumhydrid | 4,25 | 150 | 3 | 45 | 93,7 | 114 |
| 6 | Na-Ethylat | 2,5 | 190 | 30 | 45 | 79,7 | 113 |
| 7 | Dibutylzinnoxid | 0,5 | 190 | 3 | 15 | 91,3 | 115 |
| 8 | Dibutylzinnoxid | 6,0 | 190 | 3 | 21 | 86,3 | 114 |
| 9 | Dibutylzinnoxid | 10,0 | 190 | 3 | 21 | 82,4 | 117 |
| 10 | Dibutylzinnoxid | 1,25 | 190 | 10 | 21 | 96,7 | 114 |

## Patentansprüche

1. Verfahren zur Herstellung von Penthaerythrityl-tetrakis-[3-(3,5-di-t-butyl-4-hydroxyphenyl)-propionat] durch Umesterung eines $C_1$-$C_4$-Alkyl-3-(3,5-di-t-butyl -4-hydroxyphenyl)-propionats mit Pentaerythrit in Gegenwart katalytischer Mengen eines basischen Katalysators, dadurch gekennzeichnet, dass die Umesterung bei einem Ueberschuss an $C_1$-$C_4$-Alkyl-3-(3,5-di-t-butyl-4-hydroxyphenyl)-propionat von 25 bis

35 % der stöchiometrischen Menge von 4 Mol auf 1 Mol Pentaerythrit in Abwesenheit von Lösungsmitteln und bei einem Druck von 10 bis 2 mbar durchgeführt wird.

2. Verfahren gemäss Anspruch 1, dadurch gekennzeichnet, dass $C_1$-$C_4$-Alkyl Methyl bedeutet.

3. Verfahren gemäss Anspruch 1, dadurch gekennzeichnet, dass der Katalysator in einer Menge von 0,5-10 Mol %, bezogen auf das Pentaerythrit, eingesetzt wird.

4. Verfahren gemäss Anspruch 1, dadurch gekennzeichnet, dass als Katalysator Lithiumamid oder Dibutylzinnoxid in einer Menge von 1,0 bis 6 Mol %, bezogen auf das Pentaerythrit, eingesetzt wird.

5. Verfahren gemäss Anspruch 4, dadurch gekennzeichnet, dass als Katalysator Lithiumamid eingesetzt wird.

6. Verfahren gemäss Anspruch 1, dadurch gekennzeichnet, dass die Umesterung bei einer Temperatur zwischen 120 und 220°C durchgeführt wird.

## Claims

1. A process for the preparation of pentaerythrityl tetrakis-[3-(3,5-di-tert-butyl-4-hydroxyphenyl)propionate] by transesterifying a $C_1$-$C_4$alkyl 3-(3,5-di-tert-butyl-4-hydroxyphenyl)propionate with pentaerythritol, in the presence of a catalytic amount of a basic catalyst, which comprises carrying out the transesterification with an excess of $C_1$-$C_4$alkyl 3-(3,5-di-tert-butyl-4-hydroxyphenyl)propionate of 25 to 35 % of the stoichiometric amount of 4 moles per 1 mole of pentaerythritol, in the absence of a solvent at a pressure of 10 to 2 mbar.

2. A process according to claim 1, wherein $C_1$-$C_4$alkyl is methyl.

3. A process according to claim 1, wherein the catalyst is used in an amount of 0.5 to 10 mol %, based on the pentaerythritol.

4. A process according to claim 1, wherein lithium amide or dibutyltin oxide is used as catalyst in an amount of 1.0 to 6 mol %, based on the pentaerythritol.

5. A process according to claim 4, wherein lithium amide is used as catalyst.

6. A process according to claim 1, wherein the transesterification is carried out at a temperature between 120 and 220°C.

## Revendications

1. Procédé pour préparer le tétrakis-[(di-tert-butyl-3,5 hydroxy-4 phényl)-3 propionate] du pentaérythritol par transestérification d'un (di-tert-butyl-3,5 hydroxy-4 phényl)-3 propionate d'alkyle en $C_1$-$C_4$ avec le pentaérythritol, en présence de quantités catalytiques d'un catalyseur basique, procédé caractérisé en ce qu'on effectue la transestérification avec un excès d'un (di-tert-butyl-3,5 hydroxy-4 phényl)-3 propionate d'alkyle en $C_1$-$C_4$ de 25 à 35% de la quantité stoechiométrique, qui est de 4 mol pour 1 mol de pentaérythritol, en l'absence de solvent et à une pression de 10 à 2 mbar.

2. Procédé selon la revendication 1 caractérisé en ce que le radical en $C_1$-$C_4$ est le radical méthyle.

3. Procédé selon la revendication 1 caractérisé en ce que le catalyseur est mis en jeu en une quantité de 0,5 à 10% en moles par rapport au pentaérythritol.

4. Procédé selon la revendication 1 caractérisé en ce qu'on utilise, comme catalyseur, l'amidure de lithium ou l'oxyde de dibutyl-étain, en une quantité de 1,0 à 6% en moles par rapport au pentaérythritol.

5. Procédé selon la revendication 5 caractérisé en ce qu'on utilise l'amidure de lithium comme catalyseur.

6. Procédé selon la revendication 1 caractérisé en ce qu'on effectue la transestérification à une température comprise entre 120 et 220°C.